# EUROPÄISCHE PATENTSCHRIFT

(11) **EP 2 288 331 B1**
(45) Veröffentlichungstag und Bekanntmachung des Hinweises auf die Patenterteilung: **15.03.2017**
(21) Anmeldenummer: 09769139.8
(22) Anmeldetag: 15.06.2009
(51) Int. Cl.: A61K 8/34, A61K 8/73, A61Q 5/06

(54) **ZUSAMMENSETZUNGEN ZUR FORMGEBUNG KERATINISCHER FASERN AUF BASIS EINER NATÜRLICHEN, HALTGEBENDEN POLYMERKOMBINATION**
COMPOSITIONS FOR SHAPING KERATIN FIBERS BASED ON A NATURAL POLYMER COMBINATION THAT PROVIDES HOLD
COMPOSITIONS POUR METTRE EN FORME DES FIBRES KÉRATINIQUES SUR LA BASE D UNE COMBINAISON DE POLYMÈRES NATURELS DONNANT DE LA TENUE

(30) Priorität: 25.06.2008 DE 102008029848
(43) Veröffentlichungstag der Anmeldung: 02.03.2011
(73) Patentinhaber: Henkel AG & Co. KGaA, 40589 Düsseldorf (DE)
(72) Erfinder: KNAPPE, Thorsten, 22869 Schenefeld (DE); SCHEFFLER, Rene, 25479 Ellerau (DE); RICHTERS, Bernd, 21129 Hamburg (DE)
(86) Internationale Anmeldenummer: PCT/EP2009/057354
(87) Internationale Veröffentlichungsnummer: WO 2009/156290

(56) Entgegenhaltungen:
- DE-A1- 10 237 257
- JP-A- 2004 307 444
- JP-A- 2005 336 126
- US-A1- 2002 051 756
- DATABASE WPI Section Ch, Week 198305 Thomson Scientific, London, GB; Class D13, AN 1983-10801K XP002541534 T. KANAMORI, M. NAGASE: "Food comprising oil-in-water emulsion - contg. xanthan gum, guar gum and tragacanth gum, used as dressing and spread" & JP 57 206360 A (LION CORP) 17. Dezember 1982 (1982-12-17)
- DATABASE WPI Section Ch, Week 200521 Thomson Scientific, London, GB; Class A14, AN 2005-199910 XP002541535 R. IKUYAMA: "Hair cosmetics e.g. hair gel or hair dressing composition for conditioning and setting/dressing head hairs, contains vinyl pyrrolidone-hexadecane copolymer and/or vinyl pyrrolidone-eicosene copolymer and thickener" & JP 2005 053810 A (KANEBO LTD) 3. März 2005 (2005-03-03)
- ANONYMOUS: "Anders' Culinary Adventures - Home Made Hair Gel Recipe" INTERNET ARTICLE, [Online] 22. Oktober 2007 (2007-10-22), XP002541533 Gefunden im Internet: URL:http://andersau.wordpress.com/2007/01/ 15/home-made-hair-gel-recipe/> [gefunden am 2009-08-13]

## Beschreibung

Die vorliegende Offenbarung betrifft kosmetische Mittel zur temporären Umformung keratinischer Fasern, die Wasser, Xanthangummi und mindestens ein zusätzliches Polysaccharidmaterial, ausgewählt aus Tragantgummi enthält. Die vorliegende Erfindung betrifft neben der Verwendung eines solchen kosmetischen Mittels zur temporären Umformung und/oder Formfixierung keratinischer Fasern, insbesondere menschlicher Haare, ein Verfahren zur temporären Umformung keratinischer Fasern, in welchem besagtes kosmetisches Mittel Anwendung findet.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Die in Mitteln zur temporären Formgebung üblicherweise eingesetzten synthetischen Polymere werden aus entsprechenden synthetisch zugänglichen Monomeren hergestellt. Besagte Monomere werden aus fossilen Stoffen wie beispielsweise Erdöl durch Umwandlung zu den entsprechenden Polymerbausteinen u.a. unter Aufwand von Energie gewonnen.

Im Rahmen eines nachhaltigeren Umganges mit der Natur als Lebensraum und mit den Resourcen bleibt es wünschenswert, für kosmetische Produkte nur solche kosmetische Rohstoffe zu verwenden, die unter möglichst wenig Einsatz von Energie aus so genannten nachwachsenden Rohstoffen zugänglich sind. Allerdings kann eine Mengenreduktion oder gar ein Austausch besagter synthetischer Polymere nur dann vorgenommen werden, wenn der Ersatz die für den Anwendungszweck gewünschten Eigenschaften aufweist und den keratinhaltigen Fasern einen ausreichenden, stabilen Halt Form gewährleist.

Des weiteren sollen die Ersatzpolymere auf natürlicher Basis die Sprungkraft und Geschmeidigkeit der in der Form fixierten, keratinhaltigen Fasern erhalten. Die Bildung von mit dem bloßen Auge sichtbaren Polymerpartikeln auf den keratinhaltigen Fasern muss vermieden werden. Ferner darf die keratinhaltige Faser nicht stumpf wirken, sondern soll natürlich glänzen.

Aus der Druckschrift DE-A1-102 37 257 sind kosmetische Mittel zur Haarumformung bekannt, die als festigendes Polymer ein gegebenenfalls wärmebehandeltes Xanthangummi enthalten. Die Formfixierung der darin aufgeführten kosmetischen Mittel ist jedoch verbesserungswürdig.

Aufgabe der vorliegenden Erfindung war es daher, eine formfixierend wirkende, kosmetische Zusammensetzung bereitzustellen, die vorwiegend bis vollständig auf nachwachsenden Rohstoffen basiert, die eine verbesserte Formfixierung bewirkt und die vorgenannten Nachteile nicht aufweist. Dabei soll möglichst ganz auf den Einsatz der auf fossilen Rohstoffen basierenden, synthetischen Polymere verzichtet werden.

Vorliegend offenbart wird ein kosmetisches Mittel zur temporären Umformung keratinischer Fasern, insbesondere menschlicher Haare, enthaltend
(a) Wasser,
(b) Xanthangummi,
(c) mindestens ein zusätzliches Polysaccharidmaterial, ausgewählt aus der Gruppe, die gebildet wird aus Tragantgummi.

Unter keratinischen Fasern sind dabei erfindungsgemäß Pelze, Wolle, Federn und insbesondere menschliche Haare zu verstehen.

Die erfindungsgemäße Wirkstoffkombination (b) und (c) liefert in wässrigem Medium eine hervorragende festigende Wirkung auf die keratinhaltigen Fasern und eine verbesserte Filmbildung. Der resultierende Film weist die zuvor benannten Nachteile des Standes der Technik in vermindertem Masse auf.

Das kosmetische Mittel ist bevorzugt frei von synthetischen, filmbildenden Polymeren und synthetischen, festigenden Polymeren, die jeweils auf Basis von fossilen Rohstoffen hergestellt wurden. Unter fossilen Rohstoffen werden erfindungsgemäß solche Stoffe verstanden, deren Ursprung sich aus fossilen Brennstoffen herleitet.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Die kosmetischen Mittel enthalten zwingend Wasser. Der Anteil von Wasser in den Mitteln beträgt bevorzugt 10 bis 95 Gew.-%, besonders bevorzugt 30 bis 90 Gew.-%, ganz besonders bevorzugt 50 bis 80 Gew.-% - jeweils bezogen auf das Gewicht des gesamten Mittels.

Weiterhin enthalten die kosmetischen Mittel Xanthangummi. Xanthangummi ist ein natürlicher, nachwachsender Rohstoff und wird als ein anionisches Polysaccharid vom Bakterium *Xanthomonas campestris* ausgeschieden.

Das Molekülgewicht des eingesetzten Xanthangummis beträgt bevorzugt 2.10⁶ bis 20.10⁶ g/mol.

Als Molekülbausteine enthält Xanthangummi D-Glucose, D-Mannose, D-Glucuronsäure, Acetat und Pyruvat in einem etwaigen molaren Verhältnis von 28 zu 30 zu 20 zu 17 zu 5,1 bis 6,3.

Das Polymerrückgrat des Xanthangummis bildet sich aus einer Cellulose-Kette aus β-1,4-gebundenen Glucoseeinheiten.

Xanthan enthält Struktureinheiten der folgenden Formel

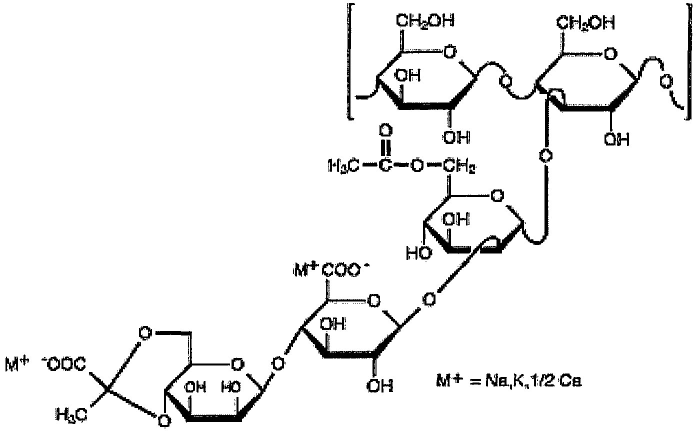

In den kosmetischen Mitteln ist das Xanthangummi bevorzugt in einer Menge von 0,1 Gew.-% bis 4 Gew.-%, insbesondere von 0,2 Gew.-% bis 2 Gew.-%, jeweils bezogen auf das Gewicht des kosmetischen Mittels, enthalten.

In einer bevorzugten Ausführungsform enthält das Mittel eine Mischung aus Xanthangummi und wärmebehandeltem Xanthangummi.

Bei Anwendung einer Mischung aus Xanthangummi und wärmebehandeltem Xanthangummi hat es sich als besonders wirksam herausgestellt, das Xanthangummi und das wärmebehandelte Xanthangummi in einem Mengenverhältnisbereich von 2 zu 1 bis 1 zu 20, insbesondere von 1 zu 2 bis 1 zu 10, einzusetzen.

Unter wärmebehandeltem Xanthangummi ist erfindungsgemäß Xanthangummi zu verstehen, welches Wärme von mindestens 40°C ausgesetzt wird. Das resultierende wärmebehandelte Xanthangummi weist eine verbesserte Dispergierbarkeit auf und lässt sich schneller in Wasser dispergieren, als Xanthangummi, das keiner Wärmebehandlung ausgesetzt wurde.

Das bevorzugt geeignete wärmebehandelte Xanthangummi weist in einer 1 Gew.-%-igen wässrigen Lösung eine Viskosität von mindestens 25000 bis 40000 mPa.s (Brookfield DV-I Viskosimeter, Spindel #6 bei 23°C und 10 U/min) auf.

Bevorzugt verwendbare, wärmebehandelte Xanthangummis liefern bei Herstellung einer 1 Gew.-%-igen, wässrigen Lösung einen pH-Wert von 4.0 bis 6.0 bei 23°C.

Das erfindungsgemäß bevorzugte wärmebehandelte Xanthangummi wurde durch temperieren von Xanthangummi bei einer Temperatur von mindestens 60°C, insbesondere von mindestens 100°C erhalten. Das Temperieren kann durch eine Vielzahl von bekannten Verfahren bewirkt werden, wie beispielsweise durch Ofen-, Fließbett-, Infrarot- oder Mikrowellenwärmebehandlung.

Im Rahmen der vorgenannten Wärmebehandlungen ist es wiederum bevorzugt, wenn das Xanthangummi vor der Wärmebehandlung einen Wassergehalt von weniger als 25 Gew.-%, insbesondere von weniger als 8 Gew.-%, ganz besonders bevorzugt von weniger als 3 Gew.-%, aufweist.

Es ist erfindungsgemäß weiterhin bevorzugt in dem Mittel solches wärmebehandeltes Xanthangummi einzusetzen, das erhalten wurde, in dem Xanthangummi mit einem Wassergehalt von weniger als 25 Gew.-% bei einer Temperatur von mindestens 60°C (insbesondere von mindestens 100°C), für mindestens 30 Minuten wärmebehandelt wird.

Es ist erfindungsgemäß besonders bevorzugt in dem Mittel solches wärmebehandeltes Xanthangummi einzusetzen, in dem Xanthangummi mit einem Wassergehalt von weniger als 8 Gew.-% bei einer Temperatur von mindestens 60°C (insbesondere von mindestens 100°C), für mindestens 30 Minuten wärmebehandelt wird.

Die bevorzugte Dauer der vorgenannten Wärmebehandlungen des Xanthangummis - insbesondere mit besagtem Wassergehalt - bei einer Temperatur von mindestens 60°C (insbesondere von mindestens 100°C) beträgt mindestens 1 Stunde.

Die besonders bevorzugte Dauer der vorgenannten Wärmebehandlungen des Xanthangummis - insbesondere mit dem bevorzugten Wassergehalt - bei einer Temperatur von mindestens 60°C (insbesondere von mindestens 100°C) beträgt mindestens 2,5 Stunden.

Daher ist es wiederum erfindungsgemäß bevorzugt in dem Mittel solches wärmebehandeltes Xanthangummi einzusetzen, das erhalten wurde, in dem Xanthangummi mit einem Wassergehalt von weniger als 25 Gew.-% bei einer Temperatur von mindestens 60°C (insbesondere von mindestens 100°C), für mindestens 1 Stunde wärmebehandelt wird.

Erfindungsgemäß weiterhin bevorzugt ist es in dem Mittel solches wärmebehandeltes Xanthangummi einzusetzen, das erhalten wurde, in dem Xanthangummi mit einem Wassergehalt von weniger als 8 Gew.-% bei einer Temperatur von mindestens 100°C, für mindestens 1 Stunde wärmebehandelt wird.

Ganz besonders bevorzugt geeignetes wärmebehandeltes Xanthangummi wird von der Firma Kelco unter dem Handelsnamen Amaze^{®} XT (mindestens 99 % Aktivsubstanz, INCI-Bezeichnung: Dehydroxanthan Gum) vertrieben.

Neben dem Xanthangummi enthält das wasserhaltige kosmetische Mittel zusätzlich mindestens ein Polysaccharidmaterial, das ausgewählt wird aus Tragantgummi.

In bevorzugter Weise enthält das Mittel das Xanthangummi in einer Menge von 0,1 Gew.-% bis 4 Gew.-%, insbesondere von 0,2 Gew.-% bis 2 Gew.-%, jeweils bezogen auf das Gewicht des kosmetischen Mittels.

Es ist erfindungsgemäß bevorzugt das Tragantgummi in dem Mittel in einer Menge von 0,2 Gew.-% bis 10 Gew.-%, insbesondere von 2 Gew.-% bis 6 Gew.-%, jeweils bezogen auf das Gewicht des kosmetischen Mittels, einzusetzen.

Ferner wurde gefunden, dass das Xanthangummi (b) und besagtes zusätzliches Polysaccharidmaterial (c) bevorzugt in einem Gewichtsverhältnisbereich von 10 zu 1 bis 1 zu 30, insbesondere von 8 zu 1 bis 1 zu 25, enthalten sind.

Tragantgummi ist ein gehärtetes Exsudat aus Stämmen und Zweigen der zu den *Astragalus-*Arten gehörenden Sträuchern, bevorzugt aus jenen von *Astragalus gummifer*, die insbesondere in verschiedenen Gegenden Vorderasiens (Türkei, Syrien) und im Iran vorkommen.

Die Einzelbausteine von Tragantgummi sind L-Rhamnose, L-Fucose, D-Xylose, L-Arabinose, D-Galactose, D-Glucose, D-Galacturonsäure, insbesondere im Verhältnis von 2,0 zu 2,8 zu 8,3 zu 24,5 zu 7,0 zu 7,6 zu 23,2. Tragant besteht zu 60 bis 70 Gew.-% aus einem in Wasser quellbaren, aber nicht löslichen Anteil (Bassorin) und zu 30 bis 40 Gew.-% aus einer wasserlöslichen Fraktion (Tragacanthinsäure).

Bassorin ist aus L-Arabinose, D-Galactose, L-Rhamnose und dem Methylester der D-Galacturonsäure aufgebaut.

Bausteine der Tragacanthinsäure (Tragacanthin, Traganthin) sind D-Galacturonsäure in der Hauptkette und D-Xylose, L-Fucose und D-Galactose in den Seitenketten.

Erfindungsgemäß einsetzbares Tragantgummi wird beispielsweise von der Firma ISP unter dem Handelsnamen ISP TG 725 (INCI-Bezeichnung: Astragalus Gummifer Gum) vertrieben.

Es ist erfindungsgemäß ganz besonders bevorzugt das Tragantgummi in dem Mittel in einer Menge von 0,1 Gew.-% bis 2 Gew.-%, insbesondere von 0,2) Gew.-% bis 1 Gew.-%, jeweils bezogen auf das Gewicht des kosmetischen Mittels, einzusetzen.

Zur Verbesserung der erfindungsgemäßen Effekte der besagten Wirkstoffkombinationen aus Xanthangummi und Tragantgummi(derivat) eignet sich bevorzugt der weitere Zusatz mindestens einer Verbindung der Formel (I),

HO-CH₂-(CHOH)ₙ-CH₂-OH (I)

worin n eine ganze Zahl von 1 bis 4 bedeutet.

Besonders effektvolle zeigen sich die Mittel, wenn sie als Verbindungen der Formel (I) Glycerin und/oder Sorbitol enthalten.

Ferner erwies sich eine Einsatzmenge der Verbindungen der Formel (I) im Bereich von 0,2 bis 10 Gew.-%, insbesondere von 0,5 bis 7 Gew.-% als vorteilhaft.

Dabei enthalten die Mittel bevorzugt mindestens eine der Kombinationen (i) bis (iv):
(i)
   - wärmebehandeltes Xanthangummi,
   - Tragantgummi,
   - Sorbitol
(ii)
   - Xanthangummi,
   - wärmebehandeltes Xanthangummi,
   - Tragantgummi,
   - Sorbitol
(iii)
   - wärmebehandeltes Xanthangummi,
   - Tragantgummi,
   - Glyzerin
(iv)
   - Xanthangummi,
   - wärmebehandeltes Xanthangummi,
   - Tragantgummi,
   - Glyzerin.

Hierbei gelten jeweils wiederum insbesondere die bevorzugten Ausführungsformen mit Blick auf das wärmebehandelte Xanthangummi und die bevorzugten Mengenangaben aller Komponenten.

Eine weitere Möglichkeit die erfindungsgemäßen Effekte der Wirkstoffkombination aus Xanthangummi mit Tragantgummi zu steigern besteht darin, dem kosmetischen Mittel zusätzlich mindestens ein Galactomannan, insbesondere Johannisbrotkernmehl und/oder Guar Gummi, zuzugeben.

Das Galactomannan des Johannisbrotkernmehls weist eine bevorzugte Molmasse von 300000 bis 360000 g/mol auf. Das Galactomannan des Johannisbrotkernmehls umfasst einer Kette aus β-(1, 4)-verknüpften D-Mannopyranosid-Einheiten, an welchen α-(1, 6)-verknüpfte α-Galactopyranosid-Einheiten haften, wobei der Gehalt Mannose/Galactose zwischen 5:1 bis 4:1 liegt.

Guargummi wird beispielsweise durch Mahlen des Endosperms der Samen des in Indien beheimateten Baumes *Cyamopsis tetragonolobus* gewonnen (35% des Samenanteiles). Der lösliche Teil ist ein nichtionogenes Polysaccharid aus β-(1, 4)-glycosidisch verknüpften D-Mannopyranose-Einheiten mit α-(1, 6)-verknüpfter D-Galactopyranose in der Seitenkette, und zwar je eine D-Galactose-Einheit auf 2 Mannose-Einheiten.

Als kosmetischen Mittel dieser Ausführungsform eignen sich bevorzugt solche, die das zusätzliche Galactomannan, insbesondere das Johannisbrotkernmehl, in einer Menge von 0,01 Gew.-% bis 0,5 Gew.-%, insbesondere von 0,05 Gew.-% bis 0,2 Gew.-%, jeweils bezogen auf das Gewicht des kosmetischen Mittels, enthalten.

Mittel, in denen bevorzugt das Guargummi als Galactomannan Einsatz findet, enthält dieses bevorzugt in einer Menge von 0,01 Gew.-% bis 0,2 Gew.-%, insbesondere von 0,05 Gew.-% bis 0,1 Gew.-%, jeweils bezogen auf das Gewicht des kosmetischen Mittels.

Es ist besonders bevorzugt, wenn das kosmetische Mittel zusätzlich als Galactomannan, insbesondere Johannisbrotkernmehl und Guargummi, insbesondere jeweils in den zuvor genannten bevorzugten Mengen, enthält.

Im Rahmen einer weiteren bevorzugten Ausführungsform enthält das Mittel zusätzlich zur erfindungsgemäßen Wirkstoffkombination aus (a), (b) und (c) (*vide supra*) Gummi Arabicum und/oder ein Derivat davon.

Gummi Arabicum ist ein getrocknetes Exsudat der Akazie. Dabei sind diejenigen Akazienarten bevorzugt geeignet, die in den tropischen und subtropischen Regionen von Afrika, Indien, Zentral- und Nordamerika vorkommen - wie beispielsweise die Arten *Acacia senegal*, *Acacia seyal*, *Acacia karroo* und *Acacia laeta* -, wobei die bedeutendste die in den südlichen Nilregionen beheimatete Art *Acacia senegal* ist.

Gummi Arabicum liegt als verzweigtes Polysaccharid vor, dessen Hauptkette aus β(1→3)-verzweigten D-Galactopyranose-Einheiten besteht. Die Hauptbestandteile als Bausteine des Gummi Arabicums sind L-Rhamnose, L-Arabinose, D-Galactose, und D-Glucuronsäure. Erfindungsgemäß bevorzugt geeignetes Gummi Arabicum weist eine Molmasse von 300000 bis 1200000 g/mol auf.

Es ist erfindungsgemäß bevorzugt das Gummi Arabicum bzw. dessen Derivate insgesamt in dem Mittel in einer Menge von 0,2 Gew.-% bis 10 Gew.-%, insbesondere von 2 Gew.-% bis 6 Gew.-%, jeweils bezogen auf das Gewicht des kosmetischen Mittels, einzusetzen. Es hat sich als bevorzugt herausgestellt, zusätzlich zu der erfindungsgemäßen Wirkstoffkombination mindestens ein nichtionisches Tensid einzusetzen. Diese Tenside können erfindungsgemäß bereits emulgierende Wirkung haben.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare oder verzweigte Fettalkohole mit 8 bis 30 Kohlenstoff-Atomen, an Fettsäuren mit 8 bis 30 Kohlenstoff-Atomen und an Alkylphenole mit 8 bis 15 C-Atomen in der Alkylgruppe,
- Anlagerungsprodukte von 2 bis 20 Einheiten Glyzerin an lineare oder verzweigte Fettalkohole mit 8 bis 30 Kohlenstoff-Atomen in der Alkylgruppe, an lineare oder verzweigte Fettsäuren mit 8 bis 30 Kohlenstoff-Atomen in der Alkylgruppe, wie beispielsweise die unter den Verkaufsbezeichnungen Dermofeel^{®} G 10 LW (Straetmans Chemische Produkte) erhältlichen Typen,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 Kohlenstoff -Atomen, an Fettsäuren mit 8 bis 30 C-Atomen und an Alkylphenole mit 8 bis 15 Kohlenstoff -Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkylolioglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3,
- Zuckertenside vom Typ der Fettsäure-N-alkylpolyhydroxyalkylamide, ein nichtionisches Tensid der Formel (E4-III), 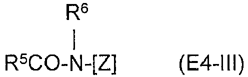 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Darunter sind wiederum solche nichtionischen Tenside besonders bevorzugt zum Einsatz in dem Mittel geeignet, die ausgewählt werden aus
- Anlagerungsprodukten von 2 bis 20 Einheiten Glyzerin an lineare oder verzweigte Fettalkohole mit 8 bis 30 Kohlenstoff-Atomen in der Alkylgruppe,
- Anlagerungsprodukten von 2 bis 20 Einheiten Glyzerin an lineare oder verzweigte Fettsäuren mit 8 bis 30 Kohlenstoff-Atomen in der Alkylgruppe,
- Zuckertensiden vom Typ der Alkyl- und Alkenyloligoglykoside gemäß obiger Formel (E4-II),
- Mischungen aus den vorgenannten Tensiden.

Die nichtionischen Tenside sind bevorzugt in einer Menge von 0,005 Gew.-% bis 10 Gew.-%, insbesondere von 0,01 bis 2 Gew.-%, jeweils bezogen auf das Gewicht des Mittels, in dem Mittel enthalten.

Ferner enthalten die erfindungsgemäßen Mittel bevorzugt zusätzlich mindestens ein Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Erfindungsgemäß sind vor allem die Extrakte aus Bambus, Leinsamen, Seerose, grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Der zusätzliche Pflanzenextrakt ist bevorzugt in dem Mittel in einer Menge von 0,05 Gew.-% bis 1,0 Gew.-%, insbesondere von 0,1 Gew.-% bis 0,5 Gew.-%, jeweils bezogen auf das Gewicht des kosmetischen Mittels, enthalten.

Es ist erfindungsgemäß bevorzugt - insbesondere wenn das Mittel als Creme formuliert ist - dass das kosmetische Mittel zusätzlich mindestens eine Ölphase enthält.

Unter einer Ölphase versteht sich erfindungsgemäß eine bei 20°C flüssige Phase, die sich bei 20°C zu weniger als 1 g in 100 g Wasser löst.

Die Ölphase besitzt bevorzugt eine Viskosität bis zu 1000 mPas, (Brookfield, RVDV II+, 20°C, 20 Umdrehungen pro Minute, Spindel Nr. 1).

In einer bevorzugten Ausführungsform wird das Öl der Ölphase ausgewählt aus mindestens einem Öl der Gruppe, die gebildet wird aus
pflanzliche Öle,
tierische Öle,
Esterölen,
flüssige Fettsäuren und/oder deren Mono,- Di- und Trifettsäureester von gesättigten und/oder ungesättigten linearen und/oder verzweigten C₆- bis C₂₂-Fettsäuren mit Glycerin.

Bevorzugte pflanzliche Öle werden ausgewählt aus mindestens einem Vertreter der Gruppe, die gebildet wird aus Amaranthöl, Sonnenblumenöl, Olivenöl, Sojaöl, Rapsöl, Ricinusöl, Sesamöl, Mandelöl, Jojobaöl, Orangenöl, Aprikosenkernöl, Macadamianussöl, Weizenkeimöl, Pfirsichkernöl und die flüssigen Anteile des Kokosöls.

Bevorzugte Esteröle werden ausgewählt aus Estern von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Bevorzugt als Öl in der Ölphase verwendbare Mono,- Di- und Trifettsäureester von gesättigten und/oder ungesättigten linearen und/oder verzweigten Fettsäuren mit Glycerin sind die insbesondere Triglyceridester aus Caprinsäure und Caprylsäure (INCI-Bezeichnung: Caprylic/Capric Triglyceride) beispielsweise erhältlich als Handelsprodukt der Firma Cognis unter der Bezeichnung Myritol^{®} 312.

Die zusätzliche Ölphase ist bevorzugt in dem Mittel in einer Menge von 0,05 Gew.-% bis 25 Gew.-%, insbesondere von 0,1 Gew.-% bis 20 Gew.-%, jeweils bezogen auf das Gewicht des kosmetischen Mittels, enthalten.

Weiterhin eignen sich Mittel, die zusätzlich mindestens einen Fettstoff enthalten.

Unter einem Fettstoff werden erfindungsgemäß solche Verbindungen verstanden, die bei 20°C zu weniger als 1 g in 100 g Wasser löslich sind.

Bevorzugt wird der Fettstoff ausgewählt aus mindestens einem Fettstoff der Gruppe, die gebildet wird aus Candelillawachs, Sheabutter, Carnaubawachs, Bienenwachs, Kokosfett, C₁₂ bis C₂₀-Fettsäuren (insbesondere Palmitinsäure, Stearinsäure)

Der zusätzliche Fettstoff ist bevorzugt in dem Mittel in einer Menge von 0,05 Gew.-% bis 35 Gew.-%, insbesondere von 1 Gew.-% bis 20 Gew.-%, jeweils bezogen auf das Gewicht des kosmetischen Mittels, enthalten.

Das kosmetische Mittel ist bevorzugt frei von synthetischen, filmbildenden Polymeren und synthetischen, festigenden Polymeren, die jeweils auf Basis von fossilen Rohstoffen hergestellt wurden. Unter fossilen Rohstoffen werden erfindungsgemäß solche Stoffe verstanden, deren Ursprung sich aus fossilen Brennstoffen ableitet.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen Polymerfilm abzuscheiden. Die filmbildenden Polymere können dabei sowohl anionisch, amphoter, nicht-ionisch, permanent kationisch oder temporär kationisch geladen sein.

Das kosmetische Mittel kann - bevorzugt unter Einhaltung der Maxime, nur solche Rohstoffe einzusetzen, die nicht ihren Ursprung in fossilen Brennstoffen haben - weiterhin zusätzliche Hilfs- und Zusatzstoffe enthalten.

Als geeignete Hilfs- und Zusatzstoffe sind insbesondere Pflegestoffe zu nennen.

Die Mittel enthalten die Komponenten (b) und (c) in einem wasserhaltigen kosmetischen Träger oder einem wässrig-alkoholischen kosmetischen Träger. Zum Zwecke der temporären Haarumformung sind solche Träger beispielsweise Lotionen, Wasser-in-Öl-Emulsionen, Öl-in-Wasser-Emulsionen, Cremes, Gele, Schäume, Pomaden, Wachse oder andere Zubereitungen, die für die Anwendung auf dem Haar geeignet sind.

Unter wässrig-alkoholischen Trägern sind im Sinne der vorliegenden Erfindung wässrige Zusammensetzungen enthaltend 3 bis 70 Gew.-% eines C₁-C₄-Alkohols, insbesondere Ethanol bzw. Isopropanol, zu verstehen. Die Mittel können zusätzlich weitere organische Lösemittel, wie beispielsweise Methoxybutanol, Benzylalkohol, Ethyldiglykol, 1,2-Propylenglykol oder 1,3-Propylenglykol enthalten. Bevorzugt sind dabei alle wasserlöslichen organischen Lösemittel.

Als Pflegestoff kann ein kationisches Tensid eingesetzt werden. Bevorzugt sind dabei kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Da sich der Zusatz oberflächenaktiver Substanzen jedoch negativ auf die hydrophoben Eigenschaften des hydrophobierten Siliciumdioxids und damit auf die Stabilität des kosmetischen Mittels auswirken kann, ist die Menge an pflegendem Tensid sorgfältig auf die Gesamtzusammensetzung abzustimmen. Vorzugsweise wird auf den Zusatz tensidischer Bestandteile verzichtet.

Als Pflegestoff eignen sich ebenfalls pflegende Polymere.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Bevorzugt sind die kationischen Proteinhydrolysate und -derivate auf pflanzlicher Basis.

Weitere erfindungsgemäß einsetzbare pflegende Polymere sind amphotere Polymere.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin B₅ (Pantothensäure, Panthenol und Pantolacton).

Als Pflegestoff eignen sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Weiterhin sind als Pflegestoff Lipide und Ölkörper, beispielsweise pflanzliche Öle, flüssige Paraffinöle, Isoparaffinöle, synthetische Kohlenwasserstoffe und Esteröle, Enzyme und Perlenextrakte geeignet.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den erfindungsgemäßen kosmetischen Mitteln zusätzlich mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Beispielhaft sei hier 2-Hydroxy-4-methoxybenzophenon-5-sulfonsäure und deren Natriumsalz (Benzophenone-4; Uvinul^{®}MS 40; Uvasorb^{®}S 5) genannt.

In einer besonderen Ausführungsform enthält das kosmetische Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Weiterhin können die kosmetischen Mittel Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-aminosäuren wie ω-Aminocapronsäure als Alkalisierungsmittel ist möglich.

Ein erster Gegenstand der Erfindung ist die Verwendung eines kosmetischen Mittels wie zuvor beschrieben zur temporären Umformung und/oder Formfixierung keratinischer Fasern, insbesondere menschlicher Haare.

Ein zweiter Gegenstand der Erfindung ist ein Verfahren zur temporären Umformung keratinischer Fasern, insbesondere menschlicher Haare, dadurch gekennzeichnet, dass ein kosmetisches Mittel wie zuvor beschrieben auf die keratinischen Fasern aufgebracht wird.

Es hat sich als bevorzugt herausgestellt, wenn die keratinischen Fasern nach der Einwirkung der kosmetischen Mittel nicht gespült und auf der Faser belassen werden.

Die nachfolgenden Beispiele sollen den Gegenstand der vorliegenden Erfindung erläutern ohne ihn in irgendeiner Weise zu beschränken.

### Beispiele

Es wurden folgende Handelsprodukte verwendet:
- Plantacare^{®} 818 UP: C8-14-Alkylpolyglucosid (ca. 51-53% Aktivsubstanzgehalt; INCI-Bezeichnung: Coco-Glucoside, Aqua (Water)) (Cognis)
- Euxyl^{®} PE 9010: Mischung aus 90 Gew.-% 2-Phenoxyethanol und 10 Gew.-% 3-(2-Ethylhexyloxy)-1,2-propandiol (100 Gew.-% Aktivsubstanz, INCI-Bezeichung: Phenoxyethanol, Ethylhexyl Glycerin) (Schülke & Mayr)
- Dermofeel^{®} G 10 LW: Decaglycerylmonolaurat (50 Gew.-% Aktivsubstanz gelöst in Wasser, HLB 15, INCI-Bezeichnung: Polyglyceryl-10 Laurate, Aqua) (Dr. Straetmans Chemische Produkte GmbH)
- Tego® Care 450: Stearylglucosid (INCI-Bezeichnung: Polyglyceryl-3 Methylglucose Distearate, HLB 11, Feststoff) (Evonik Degussa)

### 1.0 Styling Paste zum Modellieren

**Tabelle 1:**

| RG | Rohstoffe | Gew.-% |
|---|---|---|
| 1 | Wasser | 56,60 |
| 1 | Natriumhydroxid | 0,50 |
| 1 | Klettenwurzel Extrakt | 0,10 |
| 1 | Sorbitol | 2,10 |
| 1 | Plantacare^{®} 818 UP | 5,00 |
| 2 | Xanthangummi | 0,15 |
| 2 | wärmebehandeltes Xanthangummi | 1,20 |
| 2 | Tragantgummi | 0,30 |
| 2 | Stearinsäure | 4,00 |
| 2 | Capryl-/Caprinsäure-Triglycerid | 3,00 |
| 2 | Johannisbrotbaummehl | 0,10 |
| 2 | Guar Gummi | 0,05 |
| 2 | Tapioka Stärke | 0,05 |
| 2 | Palmitinsäure | 2,00 |
| 2 | Candellilawachs | 3,50 |
| 2 | Isopropylmyristat | 7,00 |
| 2 | Shea Butter | 1,00 |
| 2 | Carnaubawachs | 7,00 |
| 2 | Bienenwachs | 5,00 |
| 3 | Euxyl PE 9010^{®} | 1,00 |
| 3 | Milchsäure | 0,08 |
| 3 | Parfum | 0,50 |

### Herstellung:

### Schritt I

In einem Gefäß wurden die mit RG 1 gekennzeichneten Rohstoffe der Tabelle 1 vorgelegt. Die Mischung wurde auf 75°C unter Rühren erwärmt.

### Schritt II

In einem zweiten Gefäß wurden die mit RG 2 gekennzeichneten Rohstoffe der Tabelle 1 vorgelegt. Die Mischung wurde auf 75°C unter leichtem Rühren erwärmt.

### Schritt III

Als beide Mischungen auf 75°C temperiert waren, wurde der Inhalt des ersten Gefäßes unter starkem Rühren in das zweite Gefäß überführt. Nach Beendigung der Zugabe wurde für mindestens 15 Minuten stark weiter gerührt.

### Schritt IV

Die mit RG 3 gekennzeichneten Rohstoffe der Tabelle 1 wurden in das zweite Gefäß gegeben und die resultierende Mischung für mindestens 10 Minuten bis zur Homogenität gerührt.

Die resultierende Stylingpaste eignete sich bestens zur Separation einzelner Haarpartien. Es wurde ein Matteffekt erzielt. Die nach Anwendung der Stylingpaste bewirkte Frisur erhielt einen starken Halt und war remodulierbar.

### 2.0 Styling Creme zur Bändigung fliegender Haare

**Tabelle 2:**

| RG | Rohstoffe | Gew.-% |
|---|---|---|
| 1 | Wasser | 59,5 |
| 1 | Glycerin (pflanzlich) | 5,16 |
| 1 | Plantacare^{®} 818 UP | 4,50 |
| 2 | Shea Butter | 1,50 |
| 2 | Stearinsäure | 4,00 |
| 2 | Palmitinsäure | 4,00 |
| 2 | Sesamöl | 0,10 |
| 2 | Leinsamen Extrakt (in Sonnenblumenöl) | 0,20 |
| 2 | Candellilawachs | 2,00 |
| 2 | Isopropylmyristat | 10,00 |
| 2 | Capryl-/Caprinsäure-Triglycerid | 7,00 |
| 3 | Tragantgummi | 0,50 |
| 3 | wärmebehandeltes Xanthangummi | 0,50 |
| 4 | Euxyl PE 9010^{®} | 1,00 |
| 4 | Milchsäure | 0,04 |
| 4 | Parfum | 0,50 |

### Herstellung:

### Schritt I

In ein erstes Gefäß wurden die mit RG 2 gekennzeichneten Rohstoffe der Tabelle 2 auf 85 °C unter Rühren erwärmt, bis die Phase homogen wurde.

### Schritt II

Ein zweites Gefäß wurde mit den mit RG1 gekennzeichneten Rohstoffen der Tabelle 2 befüllt und die Mischung unter Rühren auf 60°C erwärmt. Es wurde solange gerührt, bis die Mischung homogen war und sich alle Klumpen gelöst hatten.

### Schritt III

Die mit RG 3 gekennzeichneten Rohstoffe der Tabelle 2 wurden zu der Mischung des zweiten Gefäßes hinzugefügt und unter starkem Rühren eingearbeitet. Anschließend wurde die Mischung auf 85°C erwärmt und mindestens 20 Minuten bis zur Homogenität gerührt.

### Schritt 4

Nachdem beide Mischungen auf 85°C temperiert waren, wurde die Mischung des zweiten Gefäßes zu der Mischung des ersten Gefäßes gegeben. Danach wurde auf 80°C temperiert und über einen Zeitraum von 100 Minuten gerührt.

### Schritt 5

Sodann wurde die Mischung unter Rühren auf 40°C abgekühlt und danach die mit RG 4 gekennzeichneten Rohstoffe der Tabelle 2 zugefügt. Abschließend wurde mindestens 15 Minuten bis zur Homogenität rührengelassen und dann die Mischung auf Umgebungstemperatur abkühlen gelassen.

Die resultierende Stylingcreme bändigte störrisches Haar. Die resultierende Frisur erhielt einen leichten Halt und glänzte natürlich.

### 3.0 Styling Gel

**Tabelle 3:**

| RG | Ingredient | % |
|---|---|---|
| 1 | Wasser | 78,04 |
| 2 | Milchsäure | 0,16 |
| 2 | Na-Benzoat | 0,30 |
| 2 | Ash Tree Bark Extract | 0,20 |
| 2 | Hopfen Extrakt | 0,20 |
| 2 | Bambus Extrakt | 0,20 |
| 3 | Xanthan Gummi | 1,00 |
| 3 | Gummi Arabicum | 3,00 |
| 3 | Tragantgummi | 1,00 |
| 3 | Guar Gummi | 0,30 |
| 3 | Johannisbrotbaummehl | 0,40 |
| 4 | Ethanol | 14,00 |
| 5 | Sorbitol | 0,70 |
| 5 | Tego Care 450 | 0,10 |
| 5 | Dermofeel G 10 LW | 0,10 |
| 5 | Plantacare 818 UP | 0,10 |
| 5 | Parfum | 0,10 |
| 5 | Wasser | 0,10 |

### Schritt 1

Die mit RG 1 gekennzeichneten Rohstoffe der Tabelle 3 wurden in einem ersten Gefäß unter Rühren vorgelegt.

### Schritt 2

Die mit RG 2 gekennzeichneten Rohstoffe der Tabelle 3 wurden in das erste Gefäß unter Rühren gegeben. Es wurde solange rühren gelassen, bis die Lösung homogen und klar war.

### Schritt 3

Die mit RG 3 gekennzeichneten Rohstoffe der Tabelle 3 wurden in angegebener Reihenfolge unter starkem Rühren in das erste Gefäß gegeben. Es wurde auch hier solange rühren gelassen, bis die Rohstoffe gelöst wurden und der Ansatz homogen und glatt war.

### Schritt 4

Die mit RG 4 gekennzeichneten Rohstoffe der Tabelle 3 wurden unter Rühren in das erste Gefäß gegeben. Danach wurde bis zur Homogenität rühren gelassen.

### Schritt 5

Die mit RG 5 gekennzeichneten Rohstoffe der Tabelle 3 wurden in ein zweites Gefäß geben, erwärmt und bis zur Homogenität der Mischung gerührt.

### Schritt 6

Die homogene Mischung aus dem zweiten Gefäß wurde in das erste Gefäß überführt. Die resultierende Mischung im ersten Gefäß wurde solange gerührt, bis der komplette Ansatz homogen war.

Das resultierende Stylinggel bändigte das störrische Haar des Probanden. Die resultierende Frisur erhielt einen sehr guten Halt und glänzte natürlich.

## Patentansprüche

1. Verwendung eines kosmetischen Mittels, enthaltend
(a) Wasser,
(b) Xanthangummi,
(c) mindestens ein zusätzliches Polysaccharidmaterial, ausgewählt aus der Gruppe die gebildet wird aus Tragantgummi,
zur temporären Umformung keratinischer Fasern, insbesondere menschlicher Haare.

2. Verwendung nach Anspruch 1, **dadurch gekennzeichnet, dass** es eine Mischung aus Xanthangummi und wärmebehandeltem Xanthangummi enthält, wobei das wärmebehandelte Xanthangummi erhalten wurde, indem Xanthangummi mit einem Wassergehalt von weniger als 25 Gew.-% bei einer Temperatur von mindestens 60°C, für mindestens 30 Minuten wärmebehandelt wird

3. Verwendung nach einem der Ansprüche 1 oder 2, **dadurch gekennzeichnet, dass** das Xanthangummi in einer Menge von 0,1 Gew.-% bis 4 Gew.-%, insbesondere von 0,2 Gew.-% bis 2 Gew.-%, jeweils bezogen auf das Gewicht des kosmetischen Mittels, enthalten ist.

4. Verwendung nach einem der Ansprüche 1 bis 3, **dadurch gekennzeichnet, dass** das zusätzliche Polysaccharidmaterial in einer Menge von 0,1 Gew.-% bis 10 Gew.-%, insbesondere von 0,2 Gew.-% bis 6 Gew.-%, jeweils bezogen auf das Gewicht des kosmetischen Mittels, enthalten ist.

5. Verwendung nach einem der Ansprüche 1 bis 4, **dadurch gekennzeichnet, dass** das Xanthangummi (b) und besagtes Polysaccharidmaterial (c) in einem Gewichtsverhältnisbereich von 10 zu 1 bis 1 zu 30, insbesondere von 8 zu 1 bis 1 zu 25, enthalten ist.

6. Verwendung nach einem der Ansprüche 1 bis 5, **dadurch gekennzeichnet, dass** das Xanthangummi (b) ein wärmebehandeltes Xanthangummi ist, das in einer 1 Gew.-%-igen wässrigen Lösung eine Viskosität von mindestens 25000 bis 40000 mPa.s (Brookfield DV-I Viskosimeter, Spindel #6 bei 23°C und 10 U/min) aufweist.

7. Verwendung nach einem der Ansprüche 1 bis 6, **dadurch gekennzeichnet, dass** das Xanthangummi (b) ein wärmebehandeltes Xanthangummi ist, das erhalten wurde, in dem Xanthangummi mit einem Wassergehalt von weniger als 25 Gew.-% bei einer Temperatur von mindestens 60°C (insbesondere von mindestens 100°C), für mindestens 30 Minuten wärmebehandelt wird.

8. Verwendung nach Anspruch 7, **dadurch gekennzeichnet, dass** das wärmebehandelte Xanthangummi erhalten wurde, in dem Xanthangummi mit besagtem Wassergehalt bei besagter Temperatur für mindestens 1 Stunde (insbesondere mindestens 2,5 Stunden) wärmebehandelt wird.

9. Verwendung nach einem der Ansprüche 1 bis 8, **dadurch gekennzeichnet, dass** zusätzlich mindestens eine Verbindung der Formel (I) enthalten ist,
HO-CH₂-(CHOH)ₙ-CH₂-OH (I)
worin n eine ganze Zahl von 1 bis 4 bedeutet.

10. Verwendung nach Anspruch 9, **dadurch gekennzeichnet, dass** als Verbindungen der Formel (I) Glycerin und/oder Sorbitol enthalten sind.

11. Verwendung nach einem der Ansprüche 1 bis 10, **dadurch gekennzeichnet, dass** zusätzlich mindestens ein Galactomannan, insbesondere Johannisbrotkernmehl und/oder Guar Gummi, enthalten ist.

12. Verwendung nach Anspruch 11, **dadurch gekennzeichnet, dass** das Galactomannan in einer Menge von 0,01 Gew.-% bis 0,5 Gew.-%, insbesondere von 0,05 Gew.-% bis 0,2 Gew.-%, jeweils bezogen auf das Gewicht des kosmetischen Mittels, enthalten ist.

13. Verwendung nach einem der Ansprüche 1 bis 12, **dadurch gekennzeichnet, dass** zusätzlich Gummi Arabicum, enthalten ist.

14. Verfahren zur temporären Umformung keratinischer Fasern, insbesondere menschlicher Haare, **dadurch gekennzeichnet, dass** ein kosmetisches Mittel enthaltend
(a) Wasser,
(b) Xanthangummi,
(c) mindestens ein zusätzliches Polysaccharidmaterial, ausgewählt aus der Gruppe die gebildet wird aus Tragantgummi auf die keratinischen Fasern aufgebracht wird.

## Claims

1. Use of a cosmetic agent containing
(a) water,
(b) xanthan gum, and
(c) at least one additional polysaccharide material selected from the group consisting of tragacanth gum,
for temporarily shaping keratin fibers, in particular human hair.

2. The use according to claim 1, **characterized in that** it contains a mixture of xanthan gum and heat-treated xanthan gum, the heat-treated xanthan gum being obtained by xanthan gum having a water content of less than 25 wt.% being heat treated at a temperature of at least 60 °C for at least 30 minutes.

3. The use according to either claim 1 or 2, **characterized in that** the xanthan gum is contained in an amount of from 0.1 wt.% to 4 wt.%, in particular from 0.2 wt.% to 2 wt.%, based on the weight of the cosmetic agent in each case.

4. The use according to one of claims 1 to 3, **characterized in that** the additional polysaccharide material is contained in an amount of from 0.1 wt.% to 10 wt.%, in particular from 0.2 wt.% to 6 wt.%, based on the weight of the cosmetic agent in each case.

5. The use according to one of claims 1 to 4, **characterized in that** the xanthan gum (b) and said polysaccharide material (c) are contained in a weight ratio range of from 10 : 1 to 1 : 30, in particular from 8 : 1 to 1 : 25.

6. The use according to one of claims 1 to 5, **characterized in that** the xanthan gum (b) is a heat-treated xanthan gum having a viscosity of at least from 25000 to 40000 mPas (Brookfield DV-I viscometer, spindle #6 at 23 °C and 10 rpm) in a 1 wt.% aqueous solution.

7. The use according to one of claims 1 to 6, **characterized in that** the xanthan gum (b) is a heat-treated xanthan gum obtained by xanthan gum having a water content of less than 25 wt.% being heat treated at a temperature of at least 60 °C (in particular at least 100 °C) for at least 30 minutes.

8. The use according to claim 7, **characterized in that** the heat-treated xanthan gum was obtained by xanthan gum having said water content being heat treated at said temperature for at least 1 hour (in particular at least 2.5 hours).

9. The use according to one of claims 1 to 8, **characterized in that** additionally at least one compound of formula (I),
**HO-CH₂-(CHOH)ₙ-CH₂-OH** **(I)**
is contained, in which n represents an integer from 1 to 4.

10. The use according to claim 9, **characterized in that** glycerol and/or sorbitol are contained as compounds of formula (I).

11. The use according to one of claims 1 to 10, **characterized in that** additionally at least one galactomannan, in particular carob gum and/or guar gum, is contained.

12. The use according to claim 11, **characterized in that** the galactomannan is contained in an amount of from 0.01 wt.% to 0.5 wt.%, in particular from 0.05 wt.% to 0.2 wt.%, based on the weight of the cosmetic agent in each case.

13. The use according to one of claims 1 to 12, **characterized in that** gum arabic is additionally contained.

14. A method for temporarily shaping keratin fibers, in particular human hair, **characterized in that** a cosmetic agent containing
(a) water,
(b) xanthan gum, and
(c) at least one additional polysaccharide material selected from the group consisting of tragacanth gum is applied to the keratin fibers.

## Revendications

1. Utilisation d'un agent cosmétique, contenant
(a) de l'eau,
(b) de la gomme xanthane,
(c) au moins une substance polysaccharidique supplémentaire choisie dans le groupe formé par la gomme adragante,
pour la mise en forme temporaire de fibres kératiniques, en particulier de cheveux humains.

2. Utilisation selon la revendication 1 **caractérisée en ce que** l'agent contient un mélange de gomme xanthane et de gomme xanthane soumise à un traitement thermique, la gomme xanthane soumise à un traitement thermique ayant été obtenue **en ce que** de la gomme xanthane ayant une teneur en eau inférieure à 25 % en poids à une température d'au moins 60°C est soumise à un traitement thermique pendant au moins 30 minutes.

3. Utilisation selon l'une des revendications 1 ou 2 **caractérisée en ce que** la gomme xanthane est présente dans une quantité de 0,1 % en poids à 4 % en poids, en particulier de 0,2 % en poids à 2 % en poids, par rapport au poids de l'agent cosmétique respectivement.

4. Utilisation selon l'une quelconque des revendications 1 à 3 **caractérisée en ce que** la substance polysaccharidique supplémentaire est présente dans une quantité de 0,1 % en poids à 10 % en poids, en particulier de 0,2 % en poids à 6 % en poids, par rapport au poids de l'agent cosmétique respectivement.

5. Utilisation selon l'une quelconque des revendications 1 à 4 **caractérisée en ce que** la gomme xanthane (b) et la substance polysaccharidique (c) sont présentes dans une plage de rapport pondéral de 10 sur 1 à 1 sur 30, en particulier de 8 sur 1 à 1 sur 25.

6. Utilisation selon l'une quelconque des revendications 1 à 5 **caractérisée en ce que** la gomme xanthane (b) est une gomme xanthane soumise à un traitement thermique qui, dans une solution aqueuse à 1 % en poids, présente une viscosité d'au moins 25000 à 40000 mPa·s (viscosimètre Brookfield série DV-I, broche n°6 à 23°C et 10 tr/min).

7. Utilisation selon l'une quelconque des revendications 1 à 6 **caractérisée en ce que** la gomme xanthane (b) est une gomme xanthane soumise à un traitement thermique, qui a été obtenue en ce que de la gomme xanthane ayant une teneur en eau inférieure à 25 % en poids à une température d'au moins 60°C (en particulier d'au moins 100°C) est soumise à un traitement thermique pendant au moins 30 minutes.

8. Utilisation selon la revendication 7 **caractérisée en ce que** la gomme xanthane soumise à un traitement thermique a été obtenue **en ce que** de la gomme xanthane ayant ladite teneur en eau à ladite température est soumise à un traitement thermique pendant au moins 1 heure (en particulier au moins 2,5 heures).

9. Utilisation selon l'une quelconque des revendications 1 à 8 **caractérisée en ce qu'**au moins un composé de formule (I) est présent en plus,
HO-CH₂-(CHOH)ₙ-CH₂-OH (I)
où n correspond à un nombre entier de 1 à 4.

10. Utilisation selon la revendication 9 **caractérisée en ce que** la glycérine et/ou le sorbitol sont présents en tant que composés de formule (I).

11. Utilisation selon l'une quelconque des revendications 1 à 10 **caractérisée en ce qu'**au moins un galactomannane est présent en plus, en particulier de la gomme de caroube et/ou de la gomme de guar.

12. Utilisation selon la revendication 11 **caractérisée en ce que** le galactomannane est présent dans une quantité de 0,01 % en poids à 0,5 % en poids, en particulier de 0,05 % en poids à 0,2 % en poids, par rapport au poids de l'agent cosmétique respectivement.

13. Utilisation selon l'une quelconque des revendications 1 à 12 **caractérisée en ce que** de la gomme arabique est présente en plus.

14. Procédé de mise en forme temporaire de fibres kératiniques, en particulier de cheveux humains, **caractérisé en ce qu'**un agent cosmétique contenant
(a) de l'eau,
(b) de la gomme xanthane,
(c) au moins une substance polysaccharidique supplémentaire choisie dans le groupe formé par la gomme adragante, est appliqué sur les fibres kératiniques.
